# EUROPEAN PATENT APPLICATION

(11) **EP 4 400 132 A1**
(43) Date of publication of application: **17.07.2024**
(21) Application number: 23151381.3
(22) Date of filing: 12.01.2023
(51) Int. Cl.: A61M 5/315, A61M 5/24, A61M 5/31

(54) **MULTI-DOSE SYRINGE**

(71) Applicant: Dunne, Stephen Terence, 4150-044 Porto (PT)
(72) Inventor: Dunne, Stephen Terence, 4150-044 Porto (PT)
(74) Representative: Tergau & Walkenhorst Intellectual Property GmbH

(57) **Abstract**

An accurate-dose and/or multi-dose syringe comprising an outer casing or syringe housing (127) with finger holds (131) at the proximal end and a drug delivery port (97) at the distal end capable of firmly holding a drug container (110, 100a) having a generally tubular glass or plastic barrel (111) with drug delivery means (95, 96) at the distal end of the barrel and a stopper/piston (112, 112a) disposed close to the proximal end of the barrel to contain the medicament (117) or other formulation between the stopper/piston and the drug delivery means,
where the drug delivery means (95, 96) engages with or forms a drug delivery port (97) at the distal end of the syringe housing (127) and a plunger rod (128) with finger pad (129) at one end and in communication with the cartridge stopper/piston (112, 112a) at the other end, where the plunger rod (128) has one or more radial grooves (330, 332) and one or more axial grooves (331, 333) in communication with one another and in engagement with a pin or engagement member (336) con¬nected to the syringe housing (127) where the pin travels relative to the slots as the user rotates and pushes the plunger rod (128) to prime or deliver one or more precise doses of liquid formulation via the drug delivery port (97) where the plunger rod (128) is free to rotate relative to the container stopper (112) and where the pin (336) is biased against the grooves (330, 331, 332, 333) and where the grooves have a profile that prevents the user reversing the radial movement and/or the axial movement of the plunger rod (128).

## Description

The invention relates to a syringe system, in particular an accurate-dose and/or a multi-dose manually operated syringe system.

Glass and plastic pre-filled syringes or ready-to-fill syringes are supplied for use with the drug formulation already in the syringe and ready to use. There are two formats. The first has a luer connection for attaching a hypodermic needle. The second format, the staked needle syringe, has the needle already attached. Both formats need to be lubricated with silicone oil to facilitate stopper or piston travel.

Both have advantages and disadvantages. The principal advantage of the Glass Luer type is that the silicone lubrication can be baked on giving it superior lubrication and the choice of needle is unlimited. The biggest disadvantage is that the needle needs to be attached by the user which can lead to user errors and even loss of sterility.

Glass staked needle syringes come with the needle attached and held in place which is its biggest advantage. The disadvantages are that the needle, epoxy glue and residue tungsten from its manufacture are in touch with the drug formulation during storage and that the needle tip can be damaged or blunted by the needle shield because the needle is embedded into the rubber shield to seal it. Additionally, silicone lubrication oil cannot be baked on because of the epoxy glue so staked needle pre-filled syringes must rely on inferior sprayed on silicone where the silicone can migrate during storage into the dug causing drug agglomeration or migrate between areas on the glass surface leaving areas unlubricated.

Staked needle pre-filled syringes have limited needle options and are available only in ready to fill or ready to use formats. These are expensive compared to bulk formats. These formats also need dedicated filling lines.

Vaccine drug containers are usually multi dose vials or single dose pre-filled syringes. When supplied in vials the individual doses or vaccines need to be transferred from the vial to individual disposable syringes. This is time consuming and expensive and clinical error prone. If they are supplied in pre-filled syringes each individual dose needs its own drug container and need to be individually filled on a filling line and often cold stored. Vials have the advantage that few filling lines or filling heads are required. Filling capacity is often a problem. Often also staked needle pre-filled syringes cannot be used for stability reasons so luer versions are used where the user attaches the injection needle.

An alternative to pre-filled syringes are cartridges that comprise of a glass or plastic barrel, a pierceable septum held by a crimped metal ferrule at one end and a stopper/piston at the other. A septum piercing needle or cannula is used to perforate or pierce the septum making a fluid pathway between the cartridge drug contents and the injection needle or connecting port. Such arrangements are typically used in pen injectors such as insulin pens and cartridge-based dentistry syringes.

Cartridges are available in both ready to fill and much cheaper bulk formats. They can be filled like syringes by capping, filing, and stoppering or bubble free by stoppering, filling and caping.

It is an object of the present invention to provide a multi dose syringe that can be used with various drug containers including cartridges or pre-filled syringes. The present invention both minimizes drug container numbers, storage capacity and filing capacity than single dose pre-filled syringes and reducers clinical errors relative to the use of vials and disposable syringes.

In accordance with the invention, this objective is achieved with a syringe system as defined in claim 1.

Preferred embodiments of the invention are defined in the dependent claims.

In particular a syringe system in accordance with the present invention comprises of an:
an outer casing or syringe housing with finger pads or flange or finger rings at the proximal end capable of firmly holding;
a drug container with drug delivery means at the distal end of the barrel and a stopper/piston disposed near the proximal end of the barrel to contain the medicament or other formulation between the distal and proximal ends,
a plunger rod with finger pad at one end and in communication but not attached to the cartridge stopper at the other end where the plunger rod has two or more axial grooves and two or more radial grooves in communication with one another and in engagement with a fixed pin or engagement member connected to the syringe housing and where the pin travels relative to the slots,
and where the plunger rod is free to rotate relative to the stopper.

In other words, in accordance with one aspect of the invention a guiding and engagement system is provided for the ensemble of plunger and syringe housing, determining the potential movement or displacement of plunger relative to the syringe housing. This guiding and engagement system in an aspect of the invention comprises a number of guiding grooves (in particular, both a number of radial grooves and a number of axial grooves) being in communication with one another which may be positioned either on the outer surface of the plunger or on the inner surface of the syringe housing. Corresponding to the guiding grooves, the guiding and engagement system comprises a pin or engagement member, intended to be slidingly moved in the guiding grooves and positioned at the other of plunger and syringe housing, respectively. By this system, with the pin or engagement member being in engagement with the set of grooves, the pin travels within and relative to the grooves as the user rotates and pushes the plunger rod in the syringe housing. Due to the engagement of pin in the grooves, relative motion of plunger and housing is only possible to the degree allowed by the travelling of the pin in the grooves, and therefore the system of grooves communicating with one another defines the possible motions of the plunger relative to the syringe housing. In consequence, as long as the pin is positioned in a segment of an axial groove, only axial movement of the plunger relative to the housing is allowed and possible. In contrast, as the pin is positioned in a segment of a radial groove, only rotational movement of the plunger relative to the housing is possible.

Therefore, by proper combination and sequence of the groove system provided, certain sequences of motions of the plunger within the housing may be predefined. In one preferred embodiment of the invention, a first axial groove, in which the pin is introduced first when the plunger is inserted into the syringe housing, may be assigned for priming the syringe. Since the length of the respective axial groove determines how far the plunger is allowed the be moved axially before the subsequent, radial groove is entered, the length of the first axial groove in one aspect of the invention is chosen such that the appropriate axial movement of the plunger in order to prime the syringe is allowed.

Upon entering of the pin into the crossover section of the first axial groove and the subsequent radial groove, further axial displacement of the plunger in the syringe housing is impeded. Rather, the pin can then only be moved further in rotational direction of the radial groove, i. e. by rotating the plunger in the syringe housing, until the next crossover to the subsequent axial groove is reached. From there on, further axial displacement of the plunger in the syringe housing is allowed until the pin reaches the next radial groove. Accordingly, axial displacement of the plunger again is limited by the length of the respective axiual groove. In accordance with one aspect of the invention, the length of this respective axial segment is chosen such that a precise dose of liquid formulation is delivered via the drug delivery port. In a preferred embodiment, a number of subsequent radial and axial grooves may be provided in order to allow for a sequence of several well-defined axial displacements of the plunger in the syringe housing each with exactly defined dose delivered, interrupted by the intermediate radial sections. Therefore, a reliable delivery system for multi-dose injections with high precision is achievable.

In a preferred embodiment, the pin or engagement member is biased against the grooves and the grooves have a profile that prevents the user reversing the plunger rod travel.

The drug container may be a cartridge or pre-filled syringe having a generally tubular glass or plastic barrel where the drug deliver means may be an injection needle or threaded or luer connector or a pierceable seal or septum at the distal end of the barrel.

When used with a cartridge either;
a septum needle is rigidly held in the syringe housing and is in fluid communication with a luer slip or luer lock projecting from the distal end of the syringe housing and where the cartridge is held axially in a first position and is prevented from substantially moving unaided axially forwards within the syringe housing,
or a septum needle is rigidly held in the syringe housing and is in fluid communication with an injection needle projecting from the distal end of the syringe housing and where the cartridge is held axially in a first position and is prevented from substantially moving unaided axially forwards within the syringe housing,
or a threaded fitting including means to connect an injection needle and septum needle combination projecting from the distal end of the syringe housing and where the cartridge is held axially in position and is prevented from moving axially within the syringe housing.

When used with a staked needle pre-filled syringe either;
the pre-filled syringe injection needle projects from the distal end of the syringe housing and the pre-filled syringe is held axially in position and is prevented from moving axially within the syringe housing.
and when used with a pre-filled syringe with a luer connection;
the pre-filled syringe luer connection projects from the distal end of the syringe housing and the cartridge is held axially in position and is prevented from moving axially within the syringe housing.

Note that if the injection needle is fixed, to the syringe body or to the pre-filled syringe, the syringes can generally only be used sequentially on one subject or patient. If the device has a luer or threaded connection the needle may be replaced after each dose and used with multiple patients.

The plunger rod axial grooves may be of different lengths to deliver different sized doses.

To use the syringe;
with a cartridge;
where the cartridge is free to move the user, where required, attaches an injection needle and then twists or rotates the plunger rod and then pushes the plunger rod wherein firstly the cartridge is be moved axially forward to a second position wherein the cartridge septum is perforated by the septum needle establishing fluid communication between the cartridge contents and the injection needle and further, if required, expelling any air bubble;
or where the cartridge is fixed in position the user attaches a double ended needle wherein the cartridge septum is perforated by the septum needle establishing fluid communication between the cartridge contents and the injection needle and then twists or rotates the plunger rod and then pushes the plunger rod wherein priming takes place expelling any air bubble;
with a pre-filled syringe;
the user, where required, attaches an injection needle and then twists or rotates the plunger rod and then pushes the plunger rod wherein priming takes place expelling any air bubble.

Once priming is accomplished the user further twists or rotates and the pushes the plunger rod to deliver a precise dose of drug formulation. This can be repeated multi times until all the doses are delivered. Priming might not be required and if so the first push of the plunger rod delivers the first injection dose.

Where possible the user may replace the needle after each dose is delivered.

The cartridge or pre-filled syringe may be inserted before sterilization or by the user.

The cartridge or pre-filled syringe maybe be inserted axially or radially. When inserted axially the user attaches or positions the plunger rod after inserting the cartridge. When inserted radially or sideways the plunger rod may already be in place.

The invention is generally a single use device where one container is used either supplied installed or inserted by the user. Some embodiments may be re-usable where more than one container can be used and in particular in the case of a cartridge base device where the septum needle is replaced along with the injection needle or in the case of a pre-filled syringe-based device where the needle is attached by the user. In both cases the drug containers are inserted by the user.

With a movable cartridge the cartridge may be held in the first position by a deformable or movable stop on the cartridge shoulder or cap to prevent the cartridge moving forward during transport or handling or after insertion by the user. The cartridge may be held or further held in place by friction or interference fit. The device may have a locking mechanism that holds the cartridge in place before use. If the device has a locking mechanism this needs to be unlocked so that the cartridge can be pushed to the second position by pressing the plunger rod. The lock may hold the deformable stops in the closed position that are released by activating a switch or button or other means such as removing a cap.

The device when used with a cartridge may include a cap to cover the injection needle or luer or other connection prior to use. In the case of a movable cartridge the cap may be used to lock the cartridge at the first position before use. This locking feature may be part of the cap or the housing in which case the lock is held in the locked position by the cap.

The drug container may be filled with or without an air or gas bubble. This can be expelled during priming before the first injection.

The whole assembly including with or without the drug container may be terminally sterilized in a blister or other outer packaging. Depending on the drug type and packaging and device materials the system can be sterilized using moist heat (steam), dry heat, gamma radiation or other radiation, ethylene oxide gas, vaporized hydrogen peroxide, chlorine dioxide gas, vaporized peracetic acid and nitrogen dioxide.

The drug container barrel may be made of glass or plastic such as COC or COP. The syringe system may be adapted for subcutaneous, intramuscular, intradermal, ophthalmologic, intravascular, intravenous or any other type of injections.

The current invention may be used to deliver or inject any liquid into any body including human or animal for any purpose. In one aspect of the invention, which is considered independently inventive, the syringe may by intended for being used to apply Botox to a patient. For this application, preferrably the small diameter 0,5ml pre-filled syringe, either staked needle or Luer version, may be used. The small diameter would give sufficient stroke length for feedback. The doses are around 50 ml, and the same needle may be used for one injection sequence.

In another aspect of the invention, which is considered independently inventive, the syringe may by intended for ocular, intravitreal or intraocular injections where accuracy of dose is very important. For this application, preferably the small diameter 0,5ml pre-filled syringe, either staked needle or Luer version, may be used. The small diameter would give sufficient stroke length for feedback as the dose is around 50 micro liters. For this application it is usual to give one injection with first priming the device to expel air in the system including the needle so in this application the first axial stroke would be the priming stroke and the second the very precise injection stroke. Another big advantage of the present invention is that the user cannot inadvertently reverse the plunger rod travel eliminating the chance of air being sucked into the needle and injected into the eye.

In particular the use of the invention in pre-filled syringes ("PFS"s) may be considered highly beneficial, thereby expanding the scope of possible uses for the Multi-Dose concepts. PFSs are available in smaller diameters than cartridges which increases the stroke length for a given injection and thus makes efficient use of the benefits of the present invention. In particular, current intravitreal injections use 0,5ml luer PFSs and are preferably Terminally Sterilised.

Environmentally friendly materials may be used to manufacture all or some parts of the syringe system including wood-based materials or bio-degradable plastics.

The current invention is a great improvement over the use of vials and disposable syringes as far fewer user steps are required with less likelihood of clinical errors. It also has advantages over individual dose pre-filled syringes as fewer drug containers and container fillings are required. Additionally, when a cartridge is used, the drug is only in contact with the barrel material, glass or plastic and an elastomer¬ic material. This is the same as with storage in a vial. Additionally, the cartridge may have the superior baked-on silicone.

The current invention is a great improvement over existing state of the art multi-dose deliver systems as it is of a simpler construction. By not attaching the plunger rod to the stopper the plunger rod can be rotated facilitating the multi-dose mechanism. This is generally not possible with pre-filled syringes or cartridge base devices as usually the plunger rod is firmly attached to the stopper and unable to rotate relative to the stopper.

The current invention is a multi-dose syringe system which is elegant in its simplicity and which is easy to implement.

Various embodiments of the present invention, by way of example, are provided in the figures. Therein,

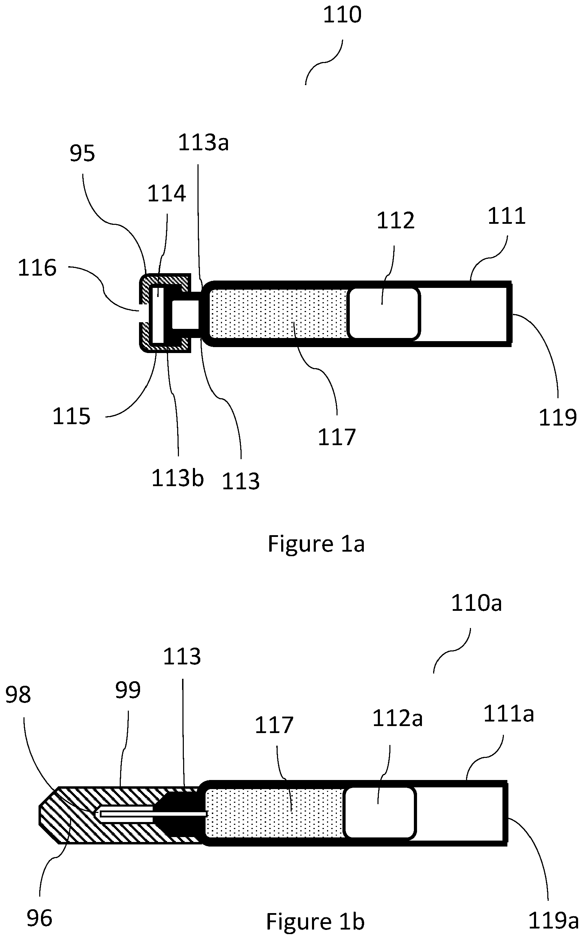

- Figure 1a: shows a 'state of the art' standard ISO 13926 Cartridge.
- Figure 1 b: shows a 'state of the art' standard ISO 11040 staked needle pre-filled syringe.
- Figure 2a: shows a single dose, cartridge based, syringe generally as per the prior art.

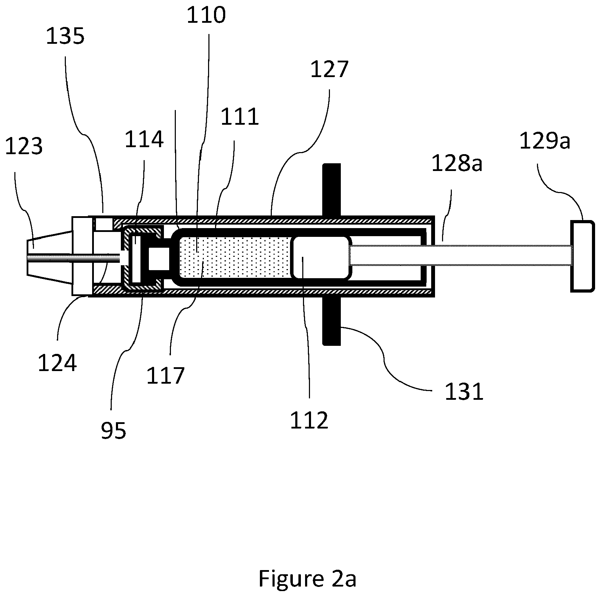

- Figure 2a: shows another single dose, cartridge based, syringe generally as per the prior art.

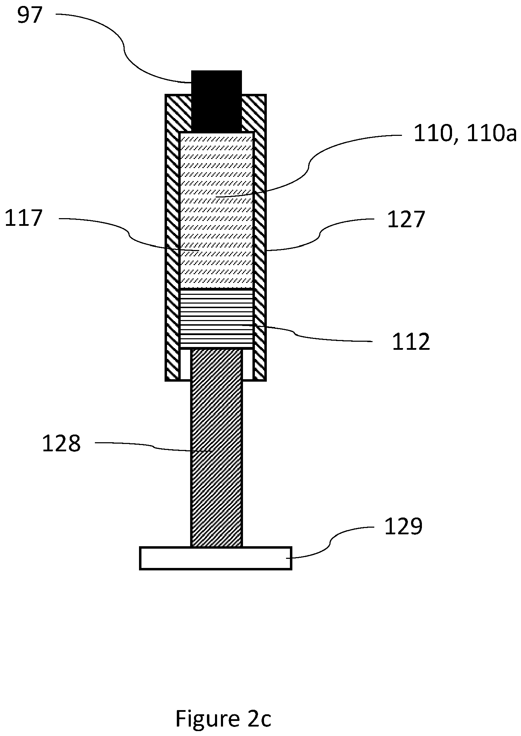

- Figure 2c: shows a schematic of the basic components of the present invention.

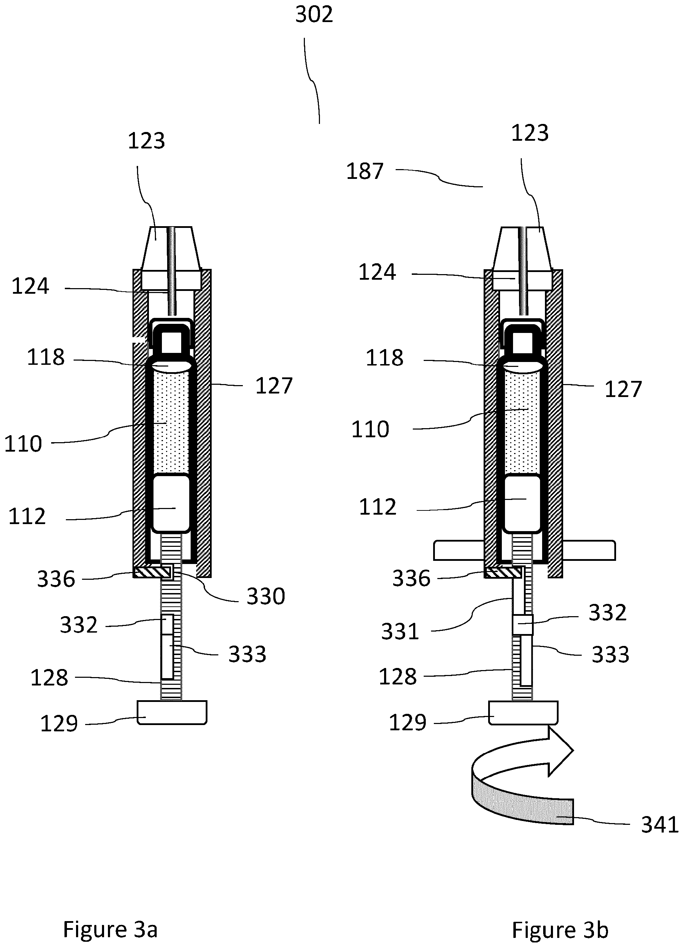

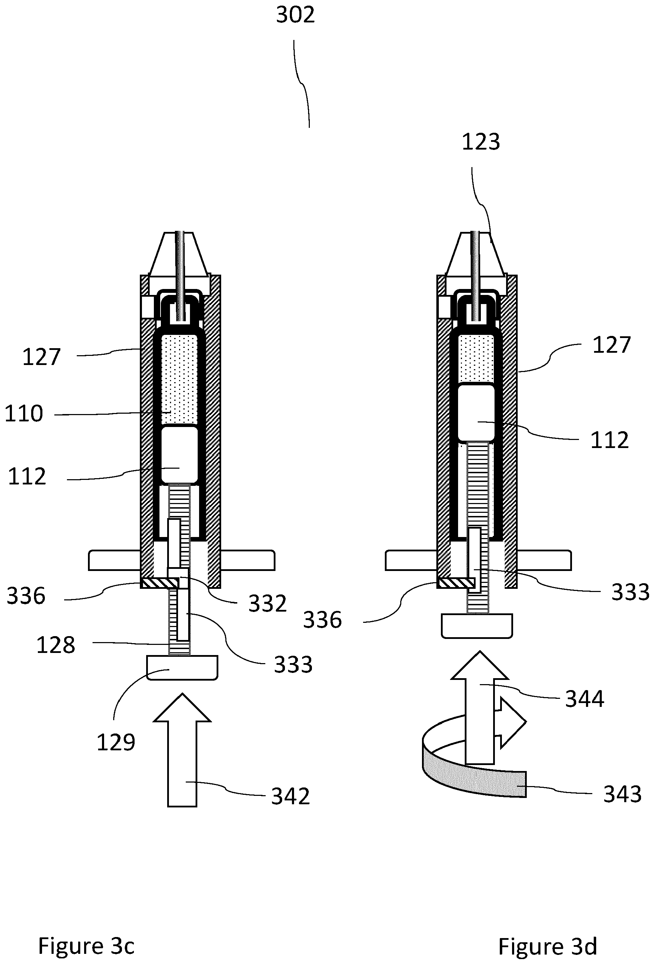

- Figures 3: show the invention where an integrated precise multi dosing mechanism is shown.

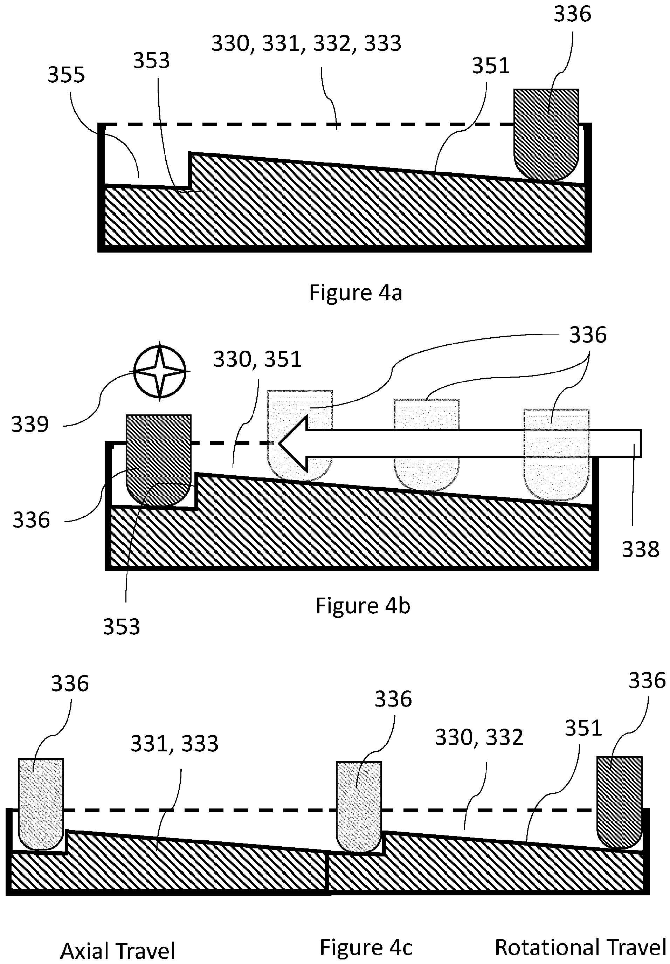

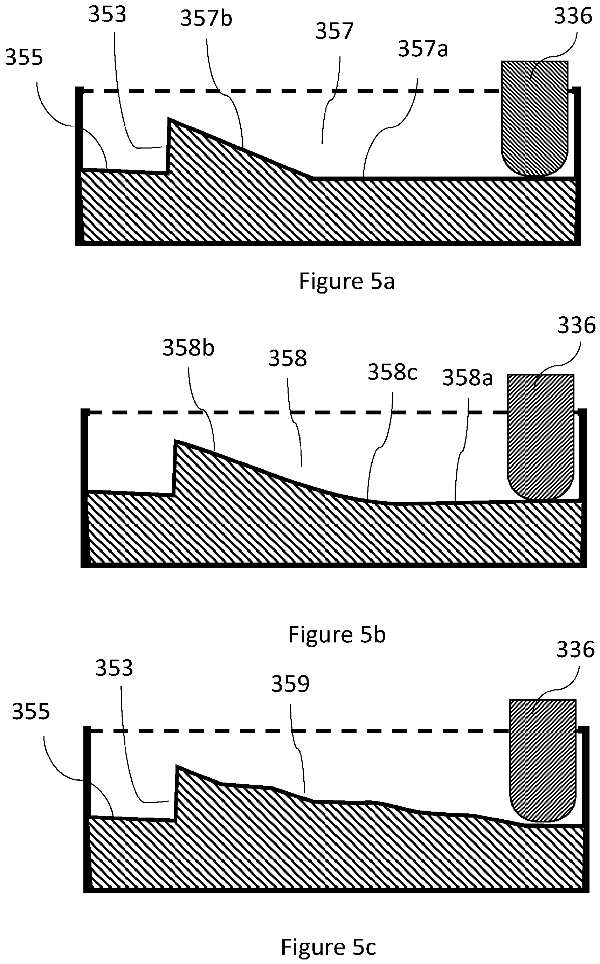

- Figures 4 and 5: show non-return plunger rod mechanisms.
- Figures 5: show possible ramp depth profiles for the grooves.

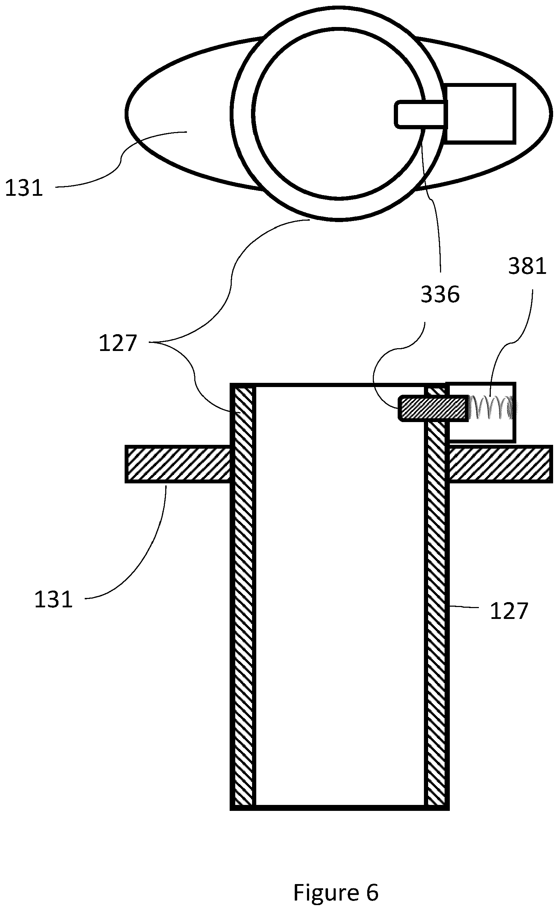

- Figure 6: shows the biased or spring-loaded pin or engagement member.

The Figures generally show the embodiment with the movable cartridge and fixed septum needle. The multi-dose mechanism is the same for the other embodiments with the fixed cartridge and fixed pre-filled syringe.

Generally identical parts are provided with the same reference numbers in all fig¬ures. The invention will be explained below primarily with reference to the embod¬iments shown. However, other embodiments are of course also conceivable and covered by the present invention.

In Figure 1a a cartridge 110 has a plastic or glass barrel 111, an open proximal end 119 and a necked 113 and flanged end 113b at a distal end forming a shoulder 113a with the barrel. A movable stopper plunger 112 at open end 119 and a rubber cap assembly 95 at the necked end or distal end 113 seal the drug formulation solution 117 within the barrel 111. Cap assembly 95 has a septum 114 which is held in place by a metal crimp or ferrule 115 which is in turn crimped to the cartridge neck 113. Ferrule 115 has a hole 116 to allow needle insertion through the septum 114.

Such cartridges are fully described in ISO 13926 and can have glass or plastic barrels.

In the following Figures the cap 95 may refer to the combination of a ferrule and a sep-tum assembly as described in Figure 1a.

In Figure 1b a pre-fillable syringe 110a has a plastic or glass barrel 111a an open proximal end 119a and a necked end 113 holding a needle assembly 96 comprising of an injection needle 98 sealed by a shield 96 at the distal end. A movable stopper plunger 112a at open proximal end 119a and the needle shield 96 at the distal end seal the drug formulation solution 117 within the barrel 111a.

In the following Figures the needle assembly 96 can refer to the combination of an injection needle 98 and needle shield 99 as described in Figure 1b or a luer fitting and sealing cap.

Figure 2a shows a basic single-dose syringe system of the prior art. A cartridge 110 is mounted within a casing or housing 127 at a first position. A luer connection 123 is located at the distal end of the syringe housing. A septum needle 124 is firmly held at the front end or distal end of housing 127 and in fluid communication with a luer or other connector 123 at the distal end of the housing 127. Cartridge 110 is axially free to move under user force in the direction of the septum needle 124 to a second position and when axially pushed by pushing plunger rod 128a at pad 129a it forces septum needle 124 to pierce the cartridge cap 95 establishing fluid communication between the cartridge liquid contents and the luer connection 123.

The user can push the plunger rod 128a using the thumb pad 129 and fin¬ger flanges 131 in the usual way to axially push the cartridge 110 to a second po¬sition to enable the syringe system and prime the system if required and perform the injection of contents 117 via luer connection 123.

Connection 123 maybe a luer slip or a luer lock for hypodermic needles or tubing.

Gas-access pathways 135 may be placed in casing 127 to facil¬itate gas sterilization of the needle assembly.

Cartridge 110 may be locked into place in the first position before use to prevent the cartridge septum being inadvertently pierced by needle 124. Alternatively, cartridge 110 may be held in the first position by friction and for instance by friction at the cartridge shoulder.

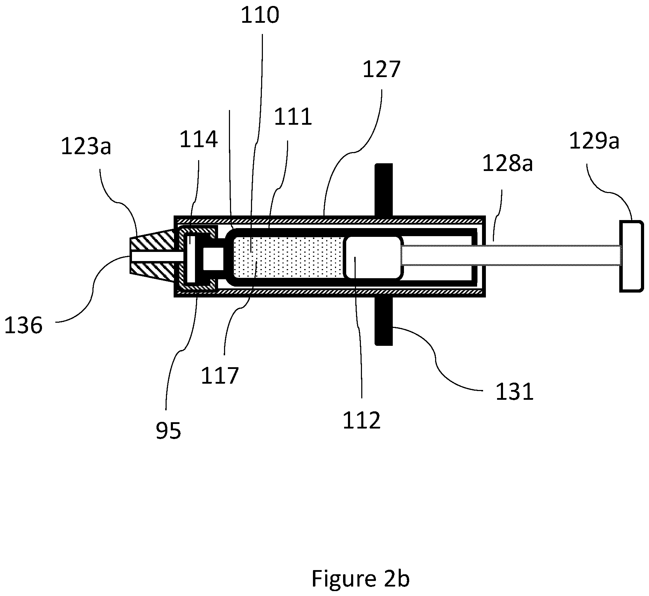

Figure 2a shows the embodiment where the cartridge 110 is movable from a first position to a second position and where the housing 127 has a septum needle 124 firmly attached. Where the cartridge 110 is fixed in position and the user attaches a double ended needle the cartridge is always fixed in the second position and a septum needle is not attached to the housing but there is a passageway to allow the double ended needle through. This is shown in Figure 2b.

In Figure 2b the luer connection has been replaced with a screw fitting 123a including a hole or passageway 136 to allow the insertion of a septum needle. Cartridge 110 is fixed into position and unable to move axially. Such an arrangement can be used with state of the art dental and/or insulin double ended needles.

Figure 2c shows a schematic of the basic parts of the present invention. A housing 127 holds a drug container 110 with contents 117. At the distal end there is a drug delivery port 97 and at the proximal end an accurate multi-dosing plunger rod 128 with finger pad 129.

The drug deliver means 95 or 96 of the drug container engages with or forms the drug delivery port 97. In the case of pre-filled syringes 110a the needle or a luer connection 96 and the delivery port 97 are one and the same. In the case of cartridges 110 that have a septum the drug deliver port 97 is formed by the engagement of the septum 114 with an injection needle or luer or other connector via a septum needle.

Figures 3 shows one embodiment of the present invention with an accurate dosing system. The embodiment shown includes the movable cartridge and fixed septum needle. Device 302 is shown before use in a first position in Figure 3a. It is shown after the first rotation of the plunger rod in Figures 3b. Figure 3c shows the device after the cartridge has been pushed for the first time and the device has been primed and figure 4d shows the device after a first dose has been delivered. The device of Figures 3 has an accurate dosing mechanism with radial grooves 330 and 332 and axial grooves 331 and 333 plus a biased pin or engagement member 336. The example shown has a priming stroke and only one dose. By adding further grooves to the plunger rod multi-doses can be delivered.

In Figures 3 spring loaded pin 336 which is attached to body 127 is firstly engaged in slot or radial groove 330 which is formed in plunger rod 128 as shown in Figure 3a. To operate plunger rod 128 is first rotated until pin 336 is lined up with axial groove 331 as shown by arrow 341 in Figure 3b. The plunger rod 128 is then pushed by the user as shown by arrow 342 until pin 336 reaches radial groove 332 and cannot move further as shown in Figure 3c. During this first axial movement of the plunger rod 128 the cartridge 110 moves forward from first position to second position and the needle 124 pierces the cartridge septum and the air bubble 118 (if present) and some liquid contents are expelled from device via luer connector 123 priming the device.

In Figure 3d the injection has taken place. To get from Figure 3c to Figure 3d first the plunger rod 128 is rotated as shown by arrow 343 so that pin 336 travels along radial groove 332 and lines up with axial groove 333. The plunger rod 128 is then pushed as shown in arrow 344. As plunger rod 128 is pushed as shown by arrow 344 it moves until pin 336 reaches the end of slot 333 and cannot move further adminis¬tering the exact dose of drug formulation determined by the length of axial groove 333.

The plunger rod may have further radial and axial slots so that more than one exact dose of drug can be administered making a multi-dose syringe. With each injection the plunger rod is rotated and then pushed. Before the injection the user may attach a new needle to the luer connector 123. When there is more than one dose more than one needle may be used especially if more than one subject is to be injected.

The first radial 330 slot is optional and is used as a safety feature to prevent premature axial plunger movement or cartridge movement.

Note that the position of the slots and pin or engagement member may be reversed with slots 330, 331, 332 and 333 formed in casing 127 with the pin 336 forming part or attached to plunger rod 128.

If the cartridge is filled without an air or a gas bubble and priming is not required, the first slot 331 can be used only for moving the cartridge forward to the second position ready for injection.

Note that in the above Figures the luer connection might be a luer slip or a luer lock for needle attachments or a line attachment and further may be a catheter tip or an eccentric tip to enable the user to attach intravenous infusion lines. It is also a feature of the present invention that if the syringe system is supplied in a sterile blister or sterile pack a nozzle cap is not essential.

The luer connection 123 can be replaced by a fixed injection needle.

In Figures 4a the grooves are shown with a depth profile. In Figure 4a groove 330, 331, 332, 333 has profile including a ramp 351, and a step 353, 355. Pin or engagement member 336 is biased towards ramp 351 and shown at the beginning of radial or axial travel along grooves 330, 331, 332 or 333. In Figure 4b arrow 338 shows the relative direction of pin 336 and groove 351 where pin or engagement member 336 is shown at the end of its travel having passed step 353. At this position the travel direction of pin 336 cannot be reversed. Cross 339 shows the future direction of travel of pin 336 at ninety degrees to groove 330 and moving from radial groove 330 to an axial groove.

In Figure 4c two separate grooves are shown. Groove 330, 332 is a radial groove while groove 331, 333 is an axial groove. There is a ninety degree turn in between the two which for simplicity is not shown in Figure 4. In Figures 4 radial groove 330 is shown opened up and flattened for clarity.

There may be two or more axial and radial grooves a pair for each dose.

Figures 5 show alternative ramp profiles. In Figure 5a the profile 357 starts as a horizontal 357a and then moves to a ramp 357b towards step 353, 355. In Figure 5b profile 358 starts as a horizontal 358a then blends into ramp 358b via a gentle curve 358c.

In Figure 4c the ramp 359 has a random profile but generally ramping up towards step 353, 355.

One further benefit or improvement of having the grooves with a profile combined with a biased pin or engagement member is that it gives feedback to the user by indicating the plunger rod is in the correct position before and after rotating or pushing it.

Figure 6 shows a spring loaded or biased engagement member 336 spring biased by coiled spring 381. Pin 381 and coiled spring 381 can be formed or molded as part of housing 127 in a single part.

## Claims

1. A syringe comprising:
an outer casing or syringe housing (127) with finger holds (131) at the proximal end and a drug delivery port (97) at the distal end capable of firmly holding;
a drug container (110, 100a) having a generally tubular glass or plastic barrel (111) with drug delivery means (95, 96) at the distal end of the barrel and a stopper/piston (112, 112a) disposed close to the proximal end of the barrel to contain the medicament (117) or other formulation between the stopper/piston and the drug delivery means,
where the drug delivery means (95, 96) engages with or forms a drug delivery port (97) at the distal end of the syringe housing (127),
a plunger rod (128) with finger pad (129) at one end and in communication with the cartridge stopper/piston (112, 112a) at the other end,
where the ensemble of plunger rod (128) and syringe housing (127) has a guiding and engagement system comprising one or more radial grooves (330, 332) and one or more axial grooves (331, 333) in communication with one another provided at one of plunger rod (128) and syringe housing (127) and in engagement with a pin or engagement member (336) connected to the other of plunger rod (128) and the syringe housing (127), respectively,
where the pin travels relative to the grooves as the user rotates and pushes the plunger rod (128) to prime or deliver one or more precise doses of liquid formulation via the drug delivery port (97),
where the plunger rod (128) is free to rotate relative to the container stopper (112) and where the grooves (330, 331, 332, 333) have a profile that prevents the user reversing the radial movement and/or the axial movement of the plunger rod (128).

2. A syringe according to claim 1 wherein the guiding and engagement system comprises at least two axial grooves (331, 333).

3. A syringe according to claim 1 or 2 where the pin (336) is biased against the grooves (330, 331, 332, 333).

4. A syringe according to any of the preceding claims where the drug container is a cartridge (110) with a pierceable septum or cap (95) where a septum needle (124) is rigidly held at the distal end of the syringe housing (127) and is in fluid communication with a luer slip or luer lock (123) with means to connect an injection needle or a transfer line and where the cartridge (110) is held in a first position and is prevented from substantially moving unaided axially forwards within the syringe housing (127),
where during the first axial movement of the plunger rod (128) the cartridge (110) is forced forwards relative to the septum needle (124) and into a second position when the septum needle perforates the cartridge septum (95, 114) establishing fluid communication between the cartridge contents (117) and the luer connector (123) and the injection needle or line.

5. A syringe according to any of the preceding claims where the drug container is a cartridge (110) with a pierceable septum or cap (95) where a septum needle (124) is rigidly held at the distal end of the syringe housing (127) and is in fluid communication with an injection needle (95) projecting from the distal end of the syringe housing and where the cartridge is held in a first position and is prevented from substantially moving unaided axially forwards within the syringe housing (127),
where during the first axial movement of the plunger rod (128) the cartridge (110) is forced forwards relative to the septum needle (124) and into a second position when the septum needle perforates the cartridge establishing fluid communication between the cartridge contents and the injection needle (95).

6. A syringe according to any of the preceding claims where the drug container is a cartridge (110) with a pierceable septum (114) or cap (95) where a threaded or other fitting projects from the distal end of the syringe housing with means to connect an injection needle and septum needle combination, and where the cartridge (110) is held axially in position,
where once the needle combination is attached to the syringe housing (127) the septum needle perforates the cartridge establishing fluid communication between the cartridge contents and the injection needle.

7. A syringe according to any of the preceding claims where the drug container is a staked needle pre-filled syringe (110a) and where the staked needle pre-filled syringe is held axially in position in the syringe housing (127) and where the staked needle (98) and needle shield (99) combination (96) project from the distal end of the syringe housing.

8. A syringe according to any of the preceding claims where the drug container is a pre-filled syringe (110a) with a luer connection and where the pre-filled syringe is held axially in position in the syringe housing (127) where the luer slip or luer lock and cap (96) project from the distal end of the syringe housing,
and where an injection needle or line can be attached to the luer connector (96).

9. A syringe according to any preceding claim where the syringe (302) is a multi-dose syringe.

10. A syringe according to any preceding claim where the syringe (302) or the syringe and drug container (110, 110a) combination are terminally sterilized inside a blister.

11. A syringe according to any preceding claim where the syringe (302) has a dose counter.

12. A syringe according to any preceding claims where the syringe (302) is re-usable and may be used with more than one drug container (110, 110a).
